# FASCICULE DE BREVET EUROPEEN

(11) **EP 2 776 648 B1**
(45) Date de publication et mention de la délivrance du brevet: **13.02.2019**
(21) Numéro de dépôt: 12806522.4
(22) Date de dépôt: 09.11.2012
(51) Int. Cl.: E04H 3/14, E04H 1/12, A61G 10/02, A61M 21/00, A63B 22/00, A61H 33/06, A61M 15/02, A61F 7/00, F24D 19/10, F24F 11/00, H05B 3/00, A61N 5/06

(54) **SALLE D'ENTRAINEMENT ET SON UTILISATION**
SCHULUNGSRAUM UND VERWENDUNG DAVON
TRAINING ROOM AND USE THEREOF

(30) Priorité: 09.11.2011 FR 1103410
(43) Date de publication de la demande: 17.09.2014
(73) Titulaire: Leclerc, Vincent, 75001 Paris (FR)
(72) Inventeur: Leclerc, Vincent, 75001 Paris (FR)
(74) Mandataire: Lejeune, Anne Claire Marie
(86) Numéro de dépôt international: PCT/FR2012/000450
(87) Numéro de publication internationale: WO 2013/068659

(56) Documents cités:
- WO-A1-02/060369
- DE-A1- 10 257 155
- DE-U1-202008 011 082

## Description

La présente invention concerne les équipements pour exercices physiques en salles. Elle concerne plus particulièrement un équipement d'entraînement permettant de modifier les conditions de réalisation d'exercices physiques, de rééducation fonctionnelle et de ce que les professionnels appellent la "réathlétisation", avec ou sans matériels, afin d'en améliorer les résultats tout en réduisant le risque de blessure musculaire, ligamentaire ou tendineuse.

L'invention vise plus spécifiquement un module constituant un volume clos, ici dénommé salle, pour la réalisation de tels objectifs.

Généralement toutes sortes d'exercices physiques et autres activités sportives, avec ou sans matériels, sont réalisés soit dans des salles où la température ambiante avoisine 20°C/22°C, soit en extérieur.

Selon l'activité pratiquée, le résultat à atteindre est obtenu en un temps imparti. Ledit résultat peut consister en une performance individuelle ou collective, ou simplement en la réalisation d'un programme, le plus souvent divisé en séquences, visant à l'entretien ou la rééducation physiques et/ou la réathlétisation.

La problématique rencontrée dans tous les cas pour l'obtention de résultats satisfaisants est :
- soit d'atteindre ce résultat en réduisant significativement le temps imparti;
- soit d'améliorer significativement ce résultat tout en gardant le même temps imparti.

Par "résultat", on entend ici indifféremment des performances recherchées, une perte de poids, une amélioration de la musculature, un rééquilibrage de la tonicité absolue ou relative des membres inférieurs et/ou supérieurs, etc.

Par ailleurs le document WO 02/060369 A1 décrit une pièce de séjour climatisée, comportant des biocapteurs pour la détermination de paramètres corporels propres à la personne qui y séjourne, puis sur cette base des moyens de commande et de régulation de paramètres climatiques et environnementaux. Dans tous les cas, la pièce de séjour ou d'exercice n'est pas close, mais simplement insolée thermiquement, et pour ce faire elle est mise en dépression de l'air ambiant.

Le document DE 102 57 155 A1 décrit un procédé et une installation pour ajuster et maintenir les teneurs en oxygène et en oxyde de carbone dans un local, et simultanément pour mettre et maintenir l'air du local en légère surpression par rapport à l'atmosphère immédiatement environnante.

Le document DE 20 2008 011082 U1 décrit une cabine de sudation/transpiration équipée d'un actionneur à pédale dont le maniement est facilité, pour que son actionnement ne nécessite pas des forces importantes.

Selon l'invention, on a maintenant conçu un équipement procurant des moyens d'entraînement aptes à apporter une solution à cette problématique.

L'invention a ainsi pour premier objet un équipement consistant en un module apte à constituer une salle d'entraînement, avec ou sans matériel pour des exercices physiques, dans laquelle ledit module se présente sous la forme d'une salle d'entraînement définissant un espace intérieur clos, ladite salle d'entraînement comportant une combinaison de:
- une pièce fermée, munie d'au moins une porte d'accès et sans fenêtre;
- des moyens pour le chauffage dudit espace intérieur par rayonnement infrarouge, avantageusement réglables par un thermostat;
- des moyens pour la régulation de l'hygrométrie ambiante dans ledit espace intérieur;
- des moyens pour l'éclairage dudit espace intérieur, de préférence avec variateur d'intensité; et
- au moins un dispositif d'aération dudit espace intérieur.

Dans des formes de réalisation avantageuses et des variantes alternatives, le dispositif selon l'invention peut comporter des caractéristiques optionnelles, qui peuvent être prises en considération séparément ou en combinaison, telles que notamment celles dans lesquelles le module comporte:
des cloisons en lames de bois ou habillées de lames de bois;
un plafond en lames de bois;
un sol intérieur composé d'un revêtement souple et amortissant;
un humidificateur, et avantageusement un dispositif pour ioniser l'air intérieur (dénommé ionisateur d'air);
un système d'éclairage à lumière variable, à commande manuelle ou asservie à un programme enregistré;
un panneau de contrôle et de commande;
un système d'aération par au moins une trappe de ventilation;
un détecteur de monoxyde de carbone; et/ou
au moins un miroir, et des équipements de confort ou d'accompagnement tels que notamment: un lecteur de CD et/ou DVD, un appareil radio, un lecteur MP3, un téléviseur ou un moniteur vidéo, etc.

Avantageusement, les moyens de régulation selon l'invention sont conçus et agencés pour pouvoir porter la température intérieure de la salle jusqu'à 45°C, et même jusqu'à 60°C, et de préférence pour maintenir la température à environ 37°C et l'hygrométrie de l'air entre environ 50 et 60% à l'intérieur de ladite salle.

L'invention sera mieux comprise, et d'autres objectifs, avantages et caractéristiques de celle-ci apparaîtront plus clairement, à la lumière de la description détaillée ci-après de modes de réalisation de l'invention.

Ces modes de réalisation sont fournis à titre purement illustratif et non-limitatif, tandis que sont annexées à la présente description des planches de dessins dans lesquelles:

la figure 1 représente schématiquement une vue en perspective schématique d'un module de salle selon l'invention, sans matériel ajouté; et

la figure 2 représente une vue de dessus en élévation du module de salle d'entraînement selon la Fig. 1, à travers un plafond supposé transparent pour permettre de situer les éléments d'équipement intérieur selon l'invention.

En référence aux dessins ainsi succinctement décrits, qui illustrent l'invention sans la limiter d'une quelconque manière, on décrit ci-après plus en détail la présente invention.

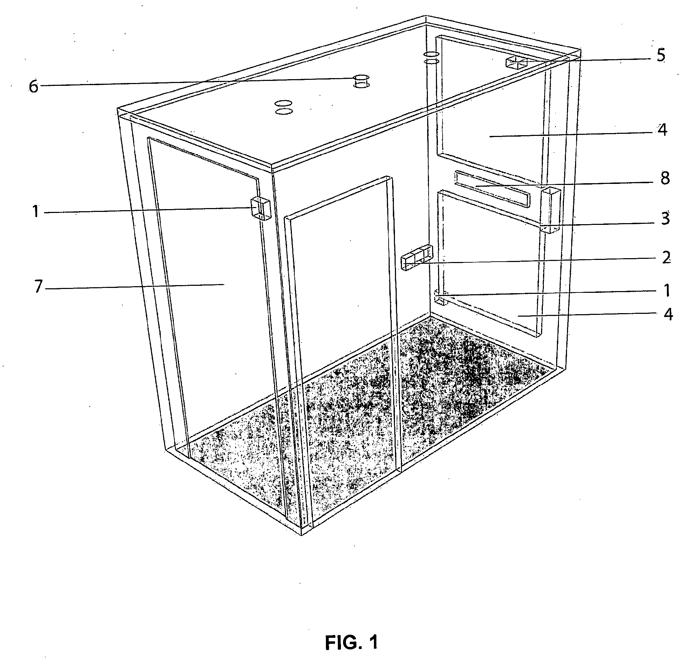

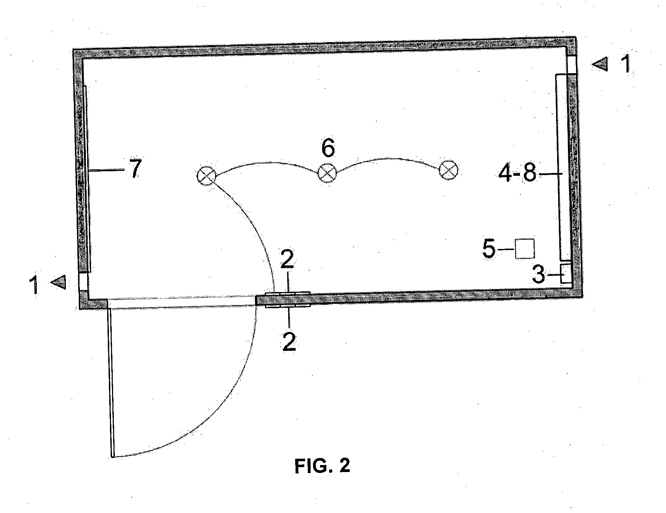

La salle d'entraînement selon l'invention peut avoir la forme parallélépipédique, sur base au sol rectangulaire, représentée sur les Figs. 1 et 2. Elle peut également prendre toute autre forme susceptible de convenir pour des applications ou des besoins de place spécifiques, par exemple avec une base au sol circulaire, hexagonale ou autre.

Ses dimensions peuvent être choisies à convenance, comme par exemple une longueur d'environ 2,60 m, une largeur d'environ 1,30 m et une hauteur d'environ 2,20 m.

Avec les formes géométriques qui le permettent, des combinaisons/juxtapositions de salles sont également possibles, voire avantageuses pour permettre un gain de place.

Dans le cas d'une pluralité n d'utilisateurs d'un même module formant salle, où n est un entier égal ou supérieur à 2, il est préconisé de donner audit module de salle des dimensions au sol représentant environ entre n et 2/3 de n fois les dimensions de base pour une personne, sans que cela soit limitatif.

La nomenclature des éléments (nécessaires ou simplement optionnels, selon les cas) apparaissant sur les dessins de la salle d'entraînement représentée, est la suivante:
- 1: trappe d'aération,
- 2: panneau de contrôle et/ou de commande (thermostat et/ou hygrostat),
- 3: humidificateur/ ionisateur d'air,
- 4: panneaux radiants infrarouge,
- 5: détecteur de monoxyde de carbone,
- 6: lampes à intensité variable,
- 7: miroir,
- 8: lecteur de CD ou DVD, radio, lecteur MP3, moniteur, téléviseur, ou autres accessoires.

Une salle d'entraînement selon l'invention consiste ainsi en un volume clos muni d'au moins une porte d'accès et sans fenêtre, comportant au moins une trappe d'aération 1, un chauffage infrarouge, par exemple par panneaux radiants 4 (qui sont au nombre de deux sur la Fig. 1), un humidificateur d'air 3 avantageusement avec hygrostat, ayant de préférence également la fonction d'ionisateur, au moins une lampe 6, et en option un panneau de commande/ contrôle 2, un détecteur de monoxyde de carbone 5, ainsi que si on le souhaite divers accessoires choisis à convenance, tels que par exemple un miroir 7 et/ou un lecteur de CD ou DVD, un appareil radio, un lecteur MP3, un téléviseur ou un moniteur vidéo, entre autres, tous référencés de manière symbolique en 8.

Le nombre des radiateurs à infrarouge n'est pas limité et ils peuvent être placés sur une ou plusieurs des parois, ou même sur ou dans le plafond.

L'invention a également pour objet l'utilisation d'une salle d'entraînement telle que décrite ci-dessus, pour procurer des moyens d'entraînement à une ou plusieurs personnes qui en ont besoin.

Dans des formes de mise en oeuvre avantageuses, l'utilisation du module de salle selon l'invention comprend:
l'ajustement de la température intérieure dans le module de salle à environ 37°C, tandis que les moyens de chauffage par infrarouge sont conçus et agencés pour pouvoir amener la température intérieure dans ladite salle jusqu'à 45°C, et même avantageusement jusqu'à 60°C.
l'ajustement du degré hygrométrique dans le module de salle entre environ 50% et 60%, de préférence à environ 55%. L'hygrostat 3 permet la mesure de l'humidité de l'air intérieur de ladite salle et agit sur l'installation de conditionnement d'air pour maintenir cette humidité.

En effet, dans sa forme d'utilisation la plus avantageuse, la salle d'entraînement selon l'invention est chauffée et maintenue à une température d'environ 37°C avec un taux d'hygrométrie compris entre 50 et 60% environ.

Une telle salle munie de ces fonctionnalités présente des atouts particulièrement remarquables pour les activités physiques qui peuvent y être exercées. En effet, la température ambiante de 37°C, associée aux exercices qui y sont pratiqués, accroît la température corporelle dès le début de l'entraînement, favorisant ainsi:
- une efficacité accrue de l'exercice et l'optimalisation de la séance d'entraînement (intensité du mouvement/fréquence de travail) pour un résultat d'une qualité supérieure comparé à celui d'une séance d'entraînement similaire effectuée dans les conditions thermiques habituelles (c'est-à-dire à 20°C/22°C environ) ou en extérieur,
- un travail de tonification et de renforcement musculaire en profondeur,
- un travail d'assouplissement musculaire et articulaire en profondeur,
- une amélioration de la protection contre le risque de blessure musculaire, ligamentaire ou tendineuse,
- une amélioration de l'efficacité des défenses de l'organisme,
- une amélioration de la circulation sanguine (vascularisation).

Le choix préféré de cette température de 37°C tient à ce que cette température, outre le fait d'être la température naturelle du corps humain, est la température à laquelle, quand elle est associée à un taux d'hygrométrie de 50% à 60%, il est encore possible de réaliser des exercices physiques générant l'aérobie et d'inspirer par la bouche ou le nez sans ressentir de gêne excessive.

A cet égard, il faut souligner que le taux d'hygrométrie a toute son importance, puisqu'il permet de maintenir une teneur en eau suffisante dans l'air, de manière à ne pas dessécher le surfactant pulmonaire (donc à maintenir une respiration aisée) et à permettre un transfert normal de l'oxygène dans le sang.

Comme on l'a indiqué plus haut, la salle d'entraînement selon l'invention est destinée à un ou plusieurs utilisateurs. Le principe d'entraînement reste le même quel que soit le nombre d'utilisateurs. L'homme du métier dispose des connaissances requises pour adapter les dimensions de la salle, ainsi que le nombre et la puissance des équipements en fonction du nombre d'utilisateurs souhaités.

La salle d'entraînement selon l'invention est particulièrement destinée à des activités d'entraînement pour les secteurs suivants:
Enseignement de disciplines sportives et d'activités de loisirs,
Santé, bien être, remise en forme et perte de poids,
Rééducation fonctionnelle et réathlétisation post-traumatique ou postopératoire à l'intention de sportifs de niveaux débutants, confirmés ou experts (sportifs de haut niveau).

Pour tous ces usages, la salle selon l'invention peut être équipée, à convenance, d'un ou plusieurs des matériels classiques en la matière, notamment un ou plusieurs appareils d'exercices, de musculation, de rééducation et/ou de réathlétisation connus de l'homme du métier ou dont la conception est à la portée de celui-ci.

## Revendications

1. Salle d'entraînement, avec ou sans matériel pour des exercices physiques, définissant un espace intérieur clos et comportant une pièce fermée, munie d'au moins une porte d'accès et sans fenêtre, **caractérisée en ce que** ladite salle d'entraînement comporte une combinaison:
• de moyens (4) pour le chauffage dudit espace intérieur par rayonnement infrarouge, avantageusement réglables par un thermostat, lesdits moyens de régulation étant conçus et agencés pour maintenir la température à environ 37°C;
• de moyens (3) pour la régulation de l'hygrométrie ambiante dans ledit espace intérieur, lesdits moyens de régulation de l'hygrométrie étant conçus et agencés pour maintenir l'hygrométrie de l'air entre environ 50 et 60% à l'intérieur de ladite salle;
• de moyens (6) pour l'éclairage dudit espace intérieur, de préférence avec variateur d'intensité; et
• d'au moins un dispositif (1) d'aération dudit espace intérieur.

2. Salle d'entraînement selon la revendication 1, **caractérisée en ce qu'**elle comporte, au moins l'un des éléments d'équipement choisis dans le groupe consistant en:
des cloisons en lames de bois;
un plafond en lames de bois;
un sol intérieur composé d'un revêtement souple et amortissant;
un humidificateur, et avantageusement un dispositif pour ioniser l'air intérieur, dénommé ionisateur d'air;
un système d'éclairage à lumière variable, à commande manuelle ou asservie à un programme enregistré;
un panneau de contrôle et de commande (2), comportant entre autres un thermostat et un hygrostat;
un système d'aération par au moins une trappe de ventilation;
un détecteur de monoxyde de carbone (5); et/ou
au moins un miroir (7), et des équipements (8) de confort ou d'accompagnement tels que notamment: un lecteur de CD et/ou DVD, un appareil radio, un lecteur MP3, un téléviseur ou un moniteur vidéo.

3. Salle d'entraînement selon la revendication 1, **caractérisée en ce que** sa base au sol est rectangulaire, et elle présente une longueur d'environ 2,60 m, une largeur d'environ 1,30 m et une hauteur d'environ 2,20 m.

4. Salle d'entraînement selon la revendication 1, **caractérisée en ce qu'**elle consiste en un volume clos muni d'au moins une porte d'accès et sans fenêtre, comportant au moins une trappe d'aération (1), un chauffage infrarouge, par exemple par panneaux radiants (4), un humidificateur d'air (3) avantageusement avec hygrostat, ayant de préférence également la fonction d'ionisateur, au moins une lampe (6), et en option un panneau de commande/contrôle (2), un détecteur de monoxyde de carbone (5), ainsi que si on le souhaite des accessoires tels que: un miroir (7) et/ou un lecteur de CD ou DVD, un appareil radio, un lecteur MP3, un téléviseur ou un moniteur vidéo (8).

5. Utilisation d'une salle d'entraînement selon l'une quelconque des revendications 1 à 4, **caractérisée en ce qu'**elle comporte:
l'ajustement de la température intérieure dans ladite salle à environ 37°C, tandis que les moyens de chauffage par infrarouge (4) sont conçus et agencés pour pouvoir amener la température intérieure dans ladite salle jusqu'à 45°C, et même avantageusement jusqu'à 60°C, et
l'ajustement du degré hygrométrique dans le module de salle entre environ 50% et 60%, de préférence à environ 55%, tandis qu'un hygrostat (3) permet la mesure de l'humidité de l'air intérieur de ladite salle et agit sur l'installation de conditionnement d'air pour maintenir ce taux d'humidité.

6. Utilisation selon la revendication 5, **caractérisée en ce que** ladite salle d'entraînement est destinée à un ou plusieurs utilisateurs, et comporte en outre des équipements et des moyens d'entraînement appropriés pour des activités d'entraînement choisies parmi:
Enseignement de disciplines sportives et d'activités de loisirs, Santé, bien être, remise en forme et perte de poids,
Rééducation fonctionnelle et réathlétisation post-traumatique ou postopératoire à l'intention de sportifs de niveaux débutants, confirmés ou experts, dénommés sportifs de haut niveau.

## Patentansprüche

1. Trainingsraum mit oder ohne Ausrüstung für Turnübungen, der einen geschlossenen Innenraum definiert und ein geschlossenes Zimmer umfasst, das mit mindestens einer Zugangstür und ohne Fenster ausgeführt ist,
**dadurch gekennzeichnet, dass** der Trainingsraum eine Kombination aus Folgendem umfasst:
- Mitteln (4) zum Heizen des Innenraums durch Infrarotstrahlung, die vorteilhafterweise durch einen Thermostat regulierbar sind, wobei die Regulierungsmittel dazu ausgeführt und vorgesehen sind, die Temperatur auf ungefähr 37°C zu halten;
- Mitteln (3) zur Regulierung der Raumhygrometrie in dem Innenraum, wobei die Mittel zur Regulierung der Hygrometrie dazu ausgeführt und vorgesehen sind, die Hygrometrie der Luft zwischen ungefähr 50 und 60% im Inneren des Raums zu halten;
- Mitteln (6) zur Beleuchtung des Innenraums, vorzugsweise mit einem Dimmer; und
- mindestens einer Vorrichtung (1) zur Belüftung des Innenraums.

2. Trainingsraum nach Anspruch 1, **dadurch gekennzeichnet, dass** er mindestens eines der Ausrüstungselemente umfasst, die in der folgenden Gruppe ausgewählt sind:
Trennwände aus Holzlamellen;
eine Decke aus Holzlamellen;
einen Innenboden, bestehend aus einer weichen und dämpfenden Auflage;
einen Luftbefeuchter, und vorteilhafterweise eine Vorrichtung zur Ionisierung der Innenluft, Luftionisator genannt;
ein Beleuchtungssystem mit Dimmer, mit manueller Steuerung oder von einem aufgezeichneten Programm gesteuert;
eine Kontroll- und Steuertafel (2), umfassend unter anderem einen Thermostat und einen Hygrostat;
ein Belüftungssystem durch mindestens eine Belüftungsklappe;
einen Kohlenmonoxid-Detektor (5); und/oder
mindestens einen Spiegel (7) und Komfort- oder Begleitausrüstungen (8), wie insbesondere: einen CD- und/oder DVD-Player, ein Radiogerät, einen MP3-Player, einen Fernseher oder einen Videorecorder.

3. Trainingsraum nach Anspruch 1, **dadurch gekennzeichnet, dass** seine Grundfläche am Boden rechteckig ist, und dass er ein Länge von ungefähr 2,60 m, eine Breite von ungefähr 1,30 m und eine Höhe von ungefähr 2,20 m aufweist.

4. Trainingsraum nach Anspruch 1, **dadurch gekennzeichnet, dass** er aus einem geschlossenen Volumen besteht, das mit mindestens einer Zugangstür und ohne Fenster ausgeführt ist, umfassend mindestens eine Belüftungsklappe (1), eine Infrarotheizung, beispielsweise mit Heizplatten (4), einen Luftbefeuchter (3), vorteilhafterweise mit Hygrostat, der vorzugsweise auch die Funktion eines Ionisators hat, mindestens eine Lampe (6) und als Option eine Steuer-/Kontrolltafel (2), einen Kohlenmonoxid-Detektor (5), sowie, falls gewünscht, Zubehör, wie beispielsweise: einen Spiegel (7) und/oder einen CD- oder DVD-Player, ein Radiogerät, einen MP3-Player, einen Fernseher oder einen Videorecorder (8) .

5. Verwendung eines Trainingsraums nach einem der Ansprüche 1 bis 4, **dadurch gekennzeichnet, dass** sie umfasst:
die Anpassung der Innentemperatur in dem Raum auf ungefähr 37°C, während die Infrarotheizmittel (4) dazu ausgeführt und vorgesehen sind, die Innentemperatur in dem Raum bis auf 45°C und vorteilhafterweise sogar bis auf 60°C zu bringen, und
die Anpassung des Hygrometriegrades in dem Raummodul zwischen ungefähr 50% und 60%, vorzugsweise auf ungefähr 55%, während ein Hygrostat (3) das Messen der Feuchtigkeit der Innenluft des Raums ermöglicht und auf die Aircondition-Anlage einwirkt, um diesen Feuchtigkeitsgrad zu halten.

6. Verwendung nach Anspruch 5, **dadurch gekennzeichnet, dass** der Trainingsraum für einen oder mehrere Benutzer bestimmt ist und ferner Trainingsausrüstungen und -mittel umfasst, die für Trainingsaktivitäten geeignet sind, ausgewählt unter:
Unterrichten von sportlichen Disziplinen und Freizeitaktivitäten,
Gesundheit, Wohlbefinden, Fitness und Gewichtsverlust,
funktioneller Rehabilitation und posttraumatischer oder post-operativer Regeneration für Sportler auf Anfänger-, Fortgeschrittenen- oder Profiebene, Spitzensportler genannt.

## Claims

1. Training room, with or without sports equipment for physical exercises, defining an enclosed indoor space and comprising a windowless closed room, provided with at least one access door, **characterized in that** said training room comprises a combination of:
• means (4) for heating said indoor space by infrared radiation, advantageously adjustable by a thermostat, said regulation means being designed and arranged to maintain the temperature at approximately 37°C;
• means (3) for the regulation of the ambient humidity in said indoor space, said humidity regulation means being designed and arranged to maintain the humidity of the air between approximately50 and 60% inside said training room;
• means (6) for lighting said indoor space, preferably with a dimmer switch; and
• at least one device (1) for ventilating said indoor space.

2. Training room according to claim 1, **characterized in that** it comprises, at least one among those elements of equipment selected from the group consisting of:
partitions made from strips of wood;
a ceiling made from strips of wood;
an inner floor composed of a flexible and shock-absorbent covering;
a humidifier, and advantageously a device for ionizing the indoor air, known as an air ionizer;
a variable lighting system with manual control or controlled according to a recorded program;
a command/control panel (2), comprising among others a thermostat and a humidistat;
a ventilation system via at least one ventilation vent;
a carbon monoxide detector (5); and/or
at least one mirror (7), and items of equipment (8) for convenience or entertainment such as in particular: a CD and/or DVD player, a radio set, an MP3 player, a television screen or a video monitor.

3. Training room according to claim 1, **characterized in that** its ground plan is rectangular, and it has a length of approximately 2.60 m, a width of approximately 1.30 m and a height of approximately 2.20 m.

4. Training room according to claim 1, **characterized in that** it consists of a windowless enclosed volume equipped with at least one access door, comprising at least one air vent (1), infrared heating, for example by radiant panels (4), an air humidifier (3) advantageously with a humidistat, preferably also having the function of ionizer, at least one lamp (6), and optionally a command/control panel (2), a carbon monoxide detector (5), as well as, if desired, accessories such as: a mirror (7) and/or a CD or DVD player, a radio set, an MP3 player, a television screen or a video monitor (8).

5. Use of a training room according to any one of claims 1 to 4, **characterized in that** it comprises:
adjusting the temperature inside said training room module to approximately 37°C, while the infrared heating means (4) are designed and arranged to be capable of raising the indoor temperature in said training room to 45°C, and even advantageously to 60°C, and
adjusting the humidity level in the training room module between approximately 50% and 60%, preferably approximately 55%, while a humidistat (3) allows measurement of the humidity of the air inside said training room and acts on the air conditioning installation to maintain this humidity level.

6. Use according to claim 5, **characterized in that** said training room is intended for one or more users, for training activities chosen from:
the teaching of sports disciplines and leisure activities, health, wellbeing, fitness and weight loss, functional rehabilitation and post-traumatic or post-operative reathletization for athletes at beginner, experienced or expert levels.
